# EUROPEAN PATENT SPECIFICATION

(11) **EP 2 830 560 B1**
(45) Date of publication and mention of the grant of the patent: **07.06.2017**
(21) Application number: 13714481.2
(22) Date of filing: 22.03.2013
(51) Int. Cl.: A61F 13/15

(54) **METHODS AND APPARATUSES FOR MAKING LEG CUFFS FOR ABSORBENT ARTICLES**
VERFAHREN UND VORRICHTUNGEN ZUR HERSTELLUNG VON BEINMANSCHETTEN FÜR SAUGFÄHIGE ARTIKEL
PROCÉDÉS ET APPAREILS POUR FABRIQUER DES MANCHONS DE JAMBE POUR ARTICLES ABSORBANTS

(30) Priority: 30.03.2012 US 201213435503
(43) Date of publication of application: 04.02.2015
(73) Proprietor: The Procter & Gamble Company, Cincinnati, OH 45202 (US)
(72) Inventor: BROWN, Tina, Cincinnati, Ohio 45241 (US); DEAN, Gregory, Hugh, West Chester, Ohio 45069 (US); SCHNEIDER, Uwe, Cincinnati, Ohio 45215 (US)
(74) Representative: Borbach, Markus
(86) International application number: PCT/US2013/033406
(87) International publication number: WO 2013/148481

(56) References cited:
- US-A- 5 622 581
- US-A1- 2007 246 152

## Description

### FIELD OF THE INVENTION

The present disclosure relates to methods for manufacturing absorbent articles, and more particularly, to methods for making leg cuffs for diapers.

### BACKGROUND OF THE INVENTION

Along an assembly line, various types of articles, such as for example, diapers and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from advancing web or webs are combined with other individual components created from other advancing web or webs. Webs of material and component parts used to manufacture diapers may include: backsheets, topsheets, leg cuffs, waist bands, absorbent core components, front and/or back ears, fastening components, and various types of elastic webs and components such as leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles.

Current diaper designs may include inner barrier leg cuffs and/or outer barrier leg cuffs. The inner barrier leg cuffs may help prevent leakage of bodily exudates, such as urine and fecal matter, and the outer barrier leg cuffs may help provide a covering over the inner barrier leg cuff to minimize the visibility of exudates through the inner barrier leg cuff. The outer barrier leg cuffs may also help provide a secondary means to capture bodily exudates that may breach the inner barrier leg cuffs. The inner barrier leg cuff may be made of various materials, such as hydrophobic nonwovens, and may be disposed on a body-facing surface of the absorbent article or may be connected to the body-facing surface of a film backsheet layer. The inner barrier leg cuff may be a substantially liquid impervious layer to help prevent bodily exudates from passing out of the sides of the absorbent article and may also be highly breathable, allowing outside air to reach the skin to help maintain a healthy level of skin hydration. Some leg cuffs may include the elastic strands, which create the contraction forces and gathers.

In some leg cuff configurations, elastic strands are bonded to substrates, such as one or more nonwoven layers. And the substrates may extend the entire length of the diaper, such as from the front waist edge to the rear waist edge. In addition, the elastic strands may not extend the full length of the diaper. In order to manufacture such a leg cuff arrangement, continuous lengths of stretched elastic strands may be intermittently bonded the continuous lengths of advancing substrates to create an elastic laminate having intermittently spaced regions where the elastics are bonded to the substrates and intermittently spaced regions where the elastics are not bonded to the substrates. In turn, the elastic laminate may be bonded to a continuous length of advancing webs, which may be assembled into a continuous length of advancing diapers. As mentioned above, the advancing webs and component parts may be subjected to a final knife cut to separate the webs into discrete diapers. The final knife cut may also be configured to cut the elastics in the laminate in regions where the elastics are not bonded to the substrates. As such, it may be intended that the severed ends of the elastics retract or snap back to the regions where the elastics are bonded to the substrates. However, in some manufacturing processes, the elastics inadvertently become bonded to other component parts of the absorbent articles during the manufacturing process. For example, in some embodiments, distal end portions of the leg cuffs may be bonded, referred to herein as "tack-down bonds," to the topsheet of the diaper in the front and/or rear waist regions. And as such, some elastics may become ensnared in the tack-down bonds, and thus, the severed elastic ends may not retract fully to the desired position on the leg cuffs. Such unretracted elastic strand ends may be detrimental to the aesthetic appearances of the diapers and/or may have a detrimental effect on leg cuff functionality.

Consequently, it would be beneficial to provide methods that are configured to create leg cuffs to help maximize the aesthetic appearance of the leg cuff when placed in an assembled product as well as help provide a more consistent manufacturing process.

### SUMMARY OF THE INVENTION

The present disclosure relates to methods for assembling absorbent articles that include leg cuff gasketing assemblies. As discussed in more detail below, each leg gasketing assembly may be formed from elastic laminates having stretched elastic strands intermittently bonded thereto. As such, the elastic laminate includes bonded regions and non-bonded regions intermittently spaced along the machine direction. The elastic strands are then intermittently deactivated by severing the strands in the non-bonded regions of the continuous elastic laminate to form continuous lengths of leg gasketing assemblies having elastic regions and deactivated regions. The continuous lengths of leg gasketing assemblies may then bonded to a continuous topsheet substrate. In some embodiments, the elastic laminate may be combined with the topsheet substrate before severing the strands in the non-bonded regions.

A method for assembling disposable absorbent articles includes the steps of: advancing a continuous topsheet substrate having a first surface and an opposing second surface in a machine direction; advancing a continuous leg cuff substrate having a first surface and an opposing second surface in the machine direction, and defining a width in a cross direction, wherein the leg cuff substrate includes opposing first and second longitudinal edges and laterally opposed first and second edge regions separated by a central region; advancing elastic strands in the machine direction in a stretched state; intermittently bonding the elastic strands in the stretched state to the first surface of the leg cuff substrate; folding the first edge region of the leg cuff substrate onto a portion of either the first edge region, the central region, or the second edge region of the leg cuff substrate such that the stretched elastic strands are positioned between the first surface of the folded first edge region and the first surface of the portion of either the first edge region, the central region, or the second edge region to form an elastic laminate, the elastic laminate including bonded regions and non-bonded regions intermittently spaced along the machine direction, wherein the elastic strands are bonded to the leg cuff substrate in the bonded regions, and wherein the elastic strands are not bonded to the leg cuff substrate in the non-bonded regions; severing elastic strands in the non-bonded regions of the elastic laminate to form a continuous length of leg gasketing assemblies having elastic regions intermittently spaced along the machine direction between deactivated regions; bonding the continuous length of leg gasketing assemblies with the continuous topsheet substrate; and cutting the continuous topsheet substrate and continuous length of leg gasketing assemblies along the cross direction in deactivated regions.

### BRIEF DESCRIPTION OF THE DRAWINGS

Figure 1 is a plan view of a diaper with a garment-facing surface oriented towards the viewer.
Figure 1A is a plan view of a diaper with a body-facing surface oriented towards the viewer.
Figure 2 is a cross-sectional view of the diaper from Figure 1 taken along line 2-2.
Figure 3 is a detailed cross-sectional view of an example of leg gasketing assemblies and a topsheet.
Figure 4 is a detailed cross-sectional view an example of a leg gasketing assembly.
Figures 5A-5P are schematic cross section views of embodiments of leg gasketing assemblies.
Figure 6 is a schematic side view of a converting apparatus adapted to manufacture leg gasketing assembles for diapers.
Figure 6A is a view of a continuous length of substrates and elastics from Figure 6 taken along line 6A-6A.

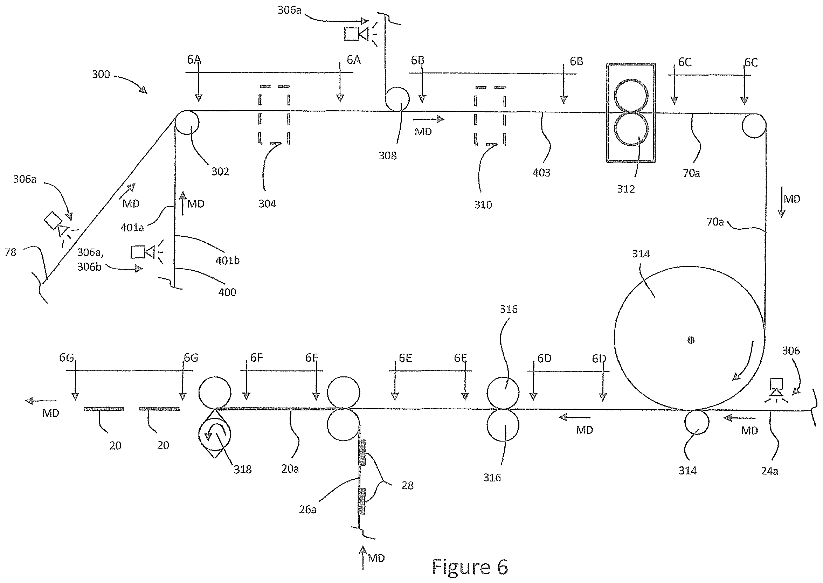

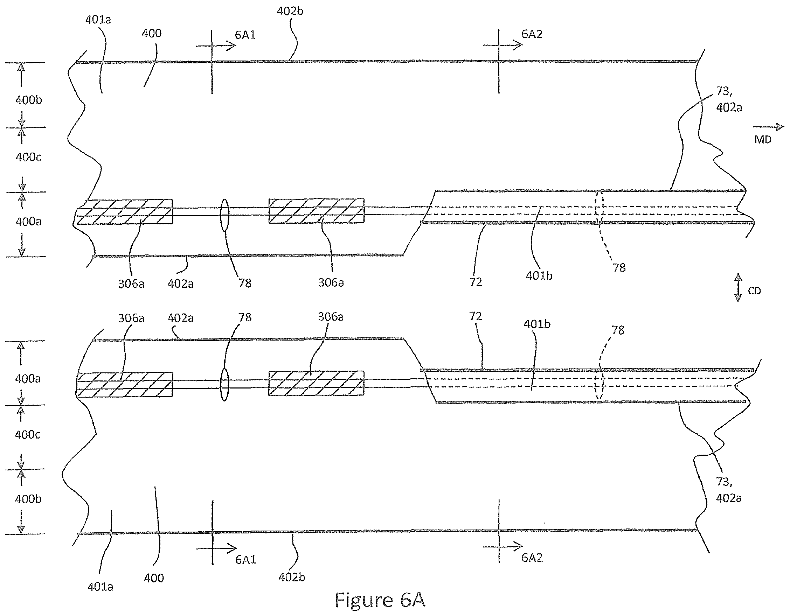

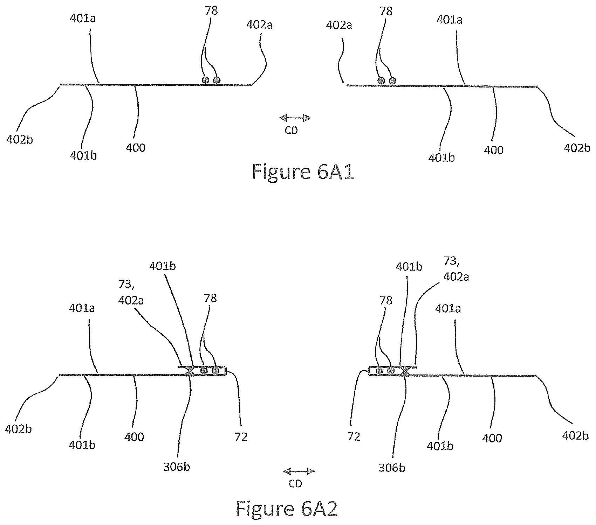

Figure 6A1 is a cross-sectional view of the substrates and elastics from Figure 6A taken along line 6A1-6A1.
Figure 6A2 is a cross-sectional view of the substrates and elastics from Figure 6A taken along line 6A2-6A2.

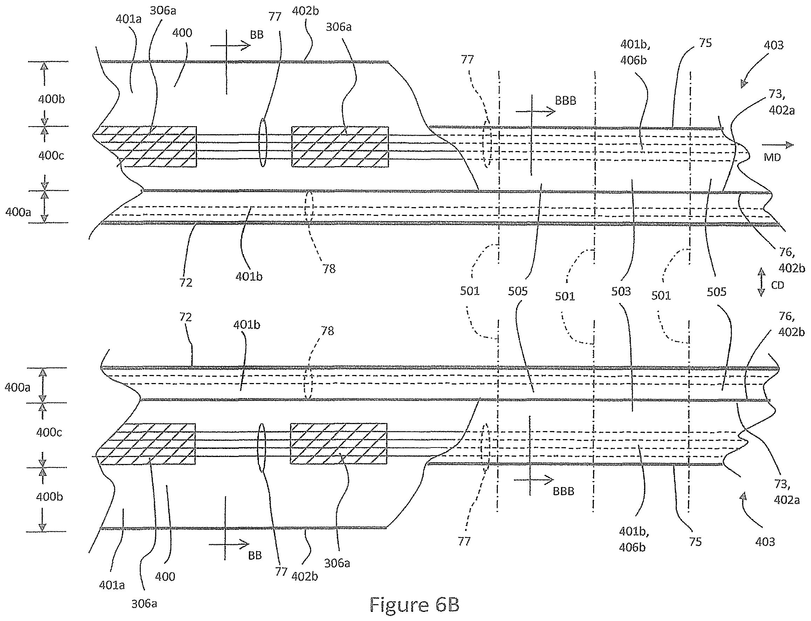

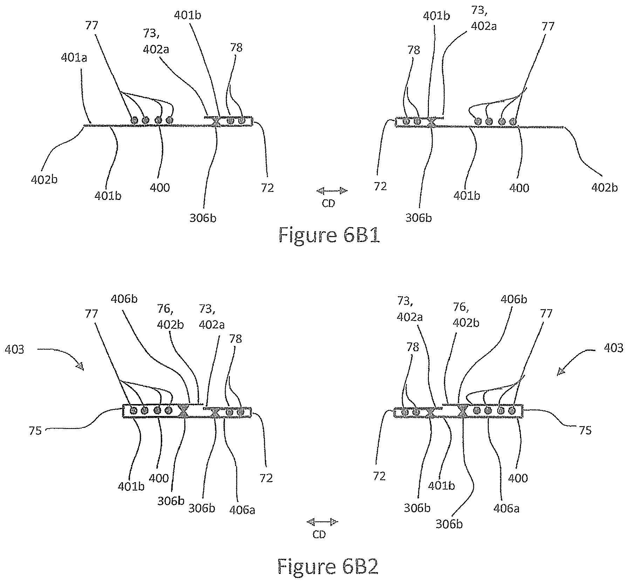

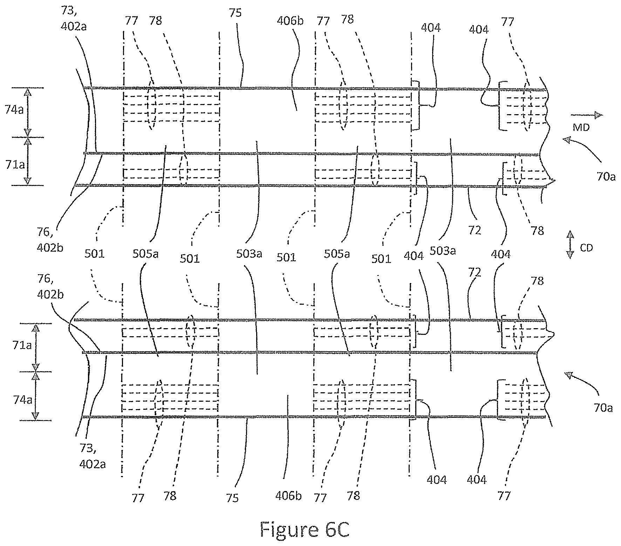

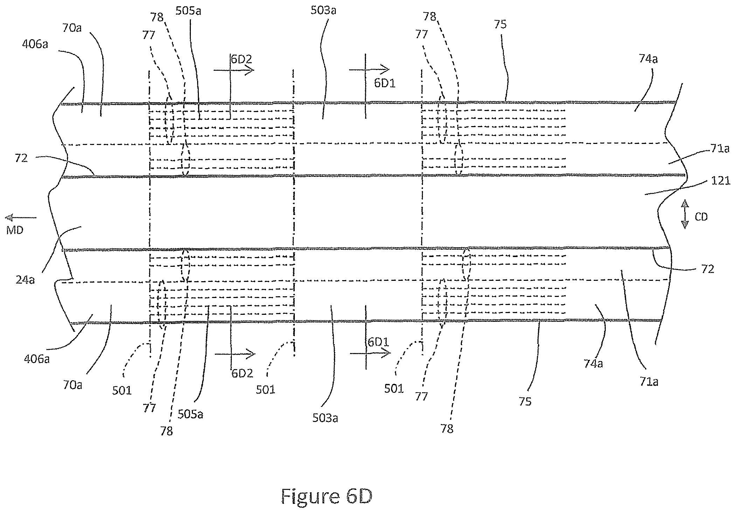

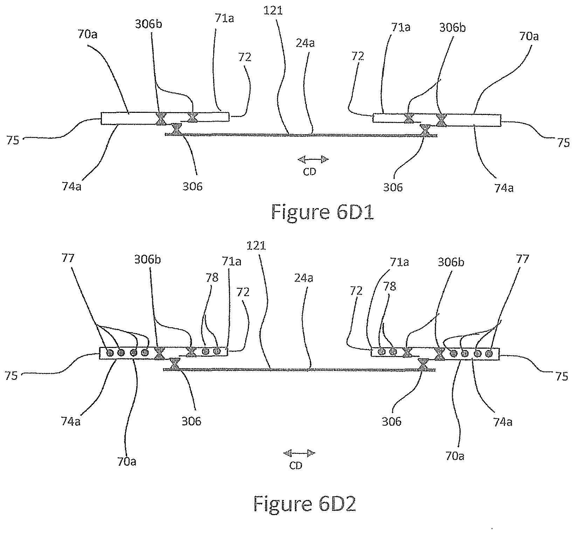

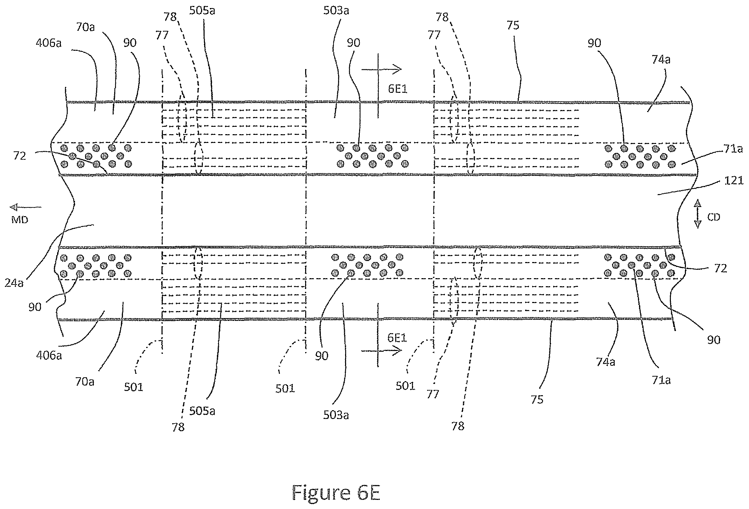

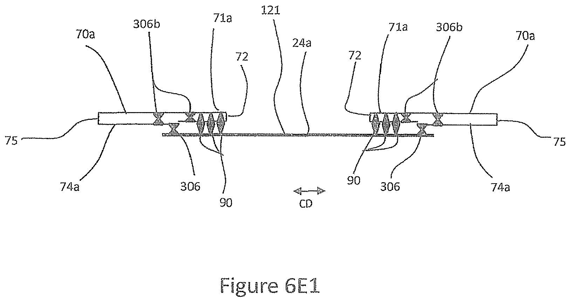

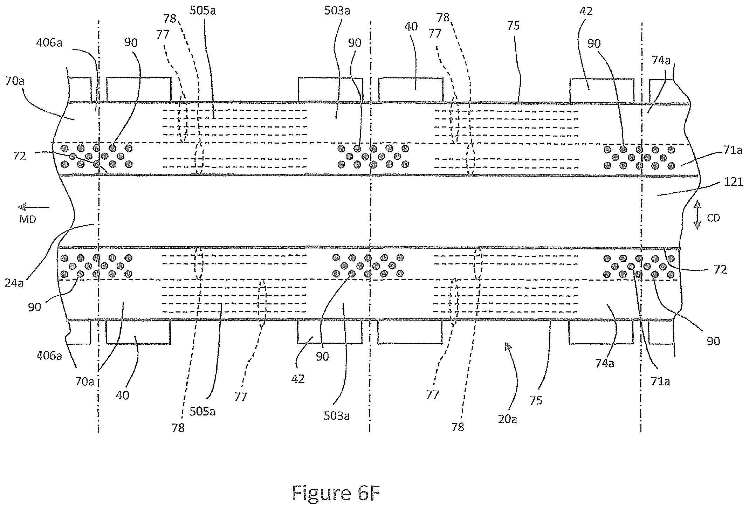

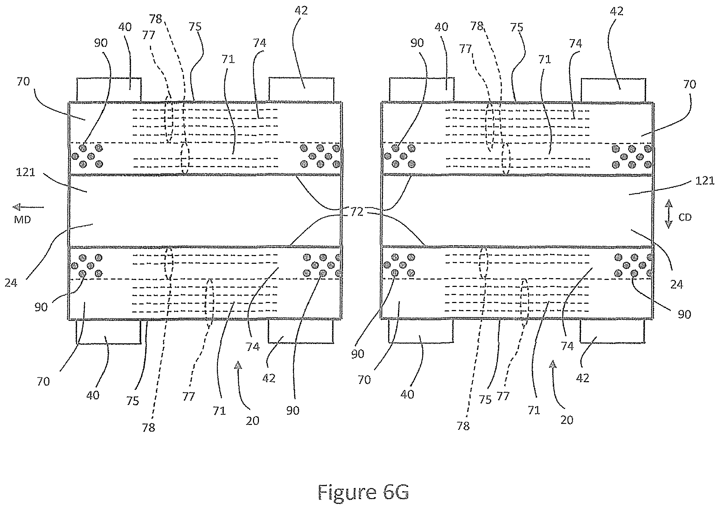

Figure 6B is a view of a continuous length of the substrates and elastics from Figure 6 taken along line 6B-6B.
Figure 6B1 is a cross-sectional view of the substrates and elastics from Figure 6B taken along line 6B1-6B1.
Figure 6B2 is a cross-sectional view of the substrates and elastics from Figure 6B taken along line 6B2-6B2.
Figure 6C is a view of a continuous length of leg gasketing assemblies from Figure 6 taken along line 6C-6C.
Figure 6D is a view of a continuous length of leg gasketing assemblies and topsheet substrate from Figure 6 taken along line 6D-6D.
Figure 6D1 is a cross-sectional view of the substrates and elastics from Figure 6D taken along line 6D1-6D1.
Figure 6D2 is a cross-sectional view of the substrates and elastics from Figure 6E taken along line 6D2-6D2.
Figure 6E is a view of a continuous length of leg gasketing assemblies and topsheet substrate with end bonds from Figure 6 taken along line 6E-6E.
Figure 6E1 is a cross-sectional view of the substrates and elastics from Figure 6E taken along line 6E1-6E1.
Figure 6F is a view of a continuous length of absorbent articles from Figure 6 taken along line 6F-6F.
Figure 6G is a view of discrete absorbent articles from Figure 6 taken along line 6G-6G.

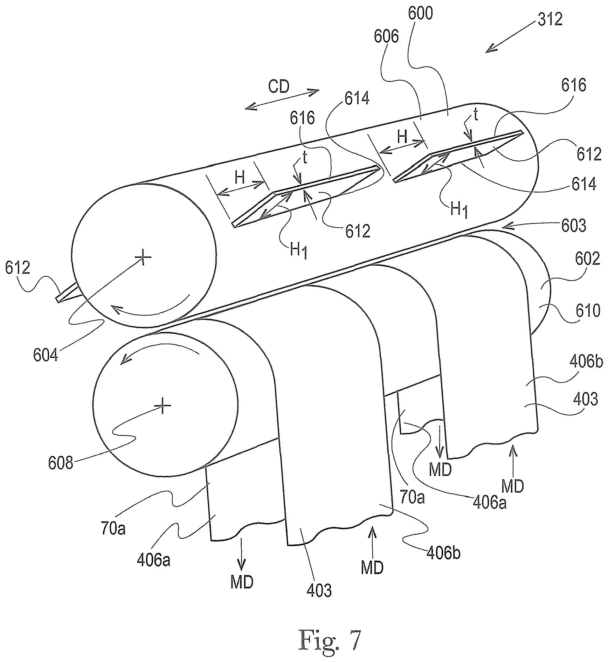

Figure 7 is a perspective view of an embodiment of a cutting apparatus.

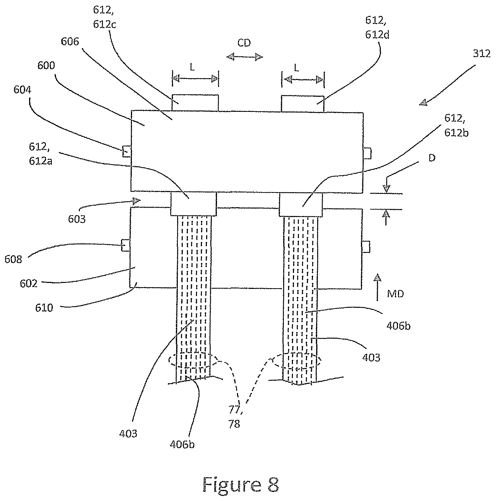

Figure 8 is a front side view of the cutting apparatus of Figure 7 as two blades are rotated toward two substrates partially wrapped around an anvil roll.

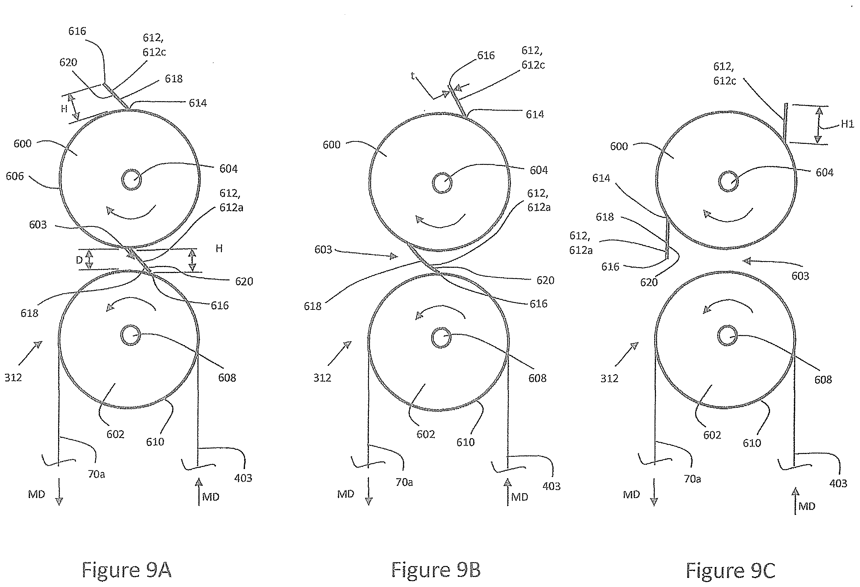

Figure 9A is a left side view of the cutting apparatus of Figure 8 showing the blade initiating contact with the substrate partially wrapped around the anvil roll.
Figure 9B shows the cutting apparatus of Figure 9A as the blade flexes while being rotated into contact with the substrate partially wrapped around the anvil roll.
Figure 9C shows the cutting apparatus of Figure 9B after the blade is rotated away from the substrate partially wrapped around the anvil roll.

### DETAILED DESCRIPTION OF THE INVENTION

The following term explanations may be useful in understanding the present disclosure:
"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (*i.e.,* element or surface is proximate to the wearer's garments that may be worn over the disposable absorbent article).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

"Longitudinal" means a direction running substantially perpendicular from a waist edge to a longitudinally opposing waist edge of an absorbent article when the article is in a flat out, uncontracted state, or from a waist edge to the bottom of the crotch, i.e. the fold line, in a bi-folded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed.

"Pre-fastened" refers herein to pant diapers manufactured and provided to consumers in a configuration wherein the front waist region and the back waist region are fastened or connected to each other as packaged, prior to being applied to the wearer. As such pant diapers may have a continuous perimeter waist opening and continuous perimeter leg openings designed for infant or adult wearers. As discussed in more detail below, a diaper pant can be preformed by various techniques including, but not limited to, joining together portions of the diaper using refastenable and/or permanent closure members (e.g., seams, heat bonds, pressure welds, adhesives, cohesive bonds, mechanical fasteners, etc.). In addition, pant diapers can be preformed anywhere along the circumference of the waist region (e.g., side fastened or connected, front waist fastened or connected, rear waist fastened or connected). U.S. Patent No. 6,120,487 provides examples of a diaper having a pre-formed waist opening and leg openings.

The present disclosure relates to methods for assembling absorbent articles, and more particularly, diapers that include leg cuff gasketing assemblies including an inner barrier leg cuff and an outer barrier leg cuff. As discussed in more detail below, each leg gasketing assembly may be formed from first and second elastic laminates having stretched elastic strands intermittently bonded thereto. An elastic laminate may be formed by intermittently bonding elastic strands between a first continuous substrate layer and a second continuous substrate layer. In some embodiments, the elastic laminate may be formed by intermittently bonding elastic strands onto portions of a single continuous substrate that are folded over the elastic strands and onto other portions of the substrate. As such, the elastic laminate includes bonded regions and non-bonded regions intermittently spaced along the machine direction, wherein the elastic strands are bonded to the substrate in the bonded regions, and wherein the elastic strands are not bonded to the substrate substrate in the non-bonded regions. The elastic strands are then intermittently deactivated by severing the strands in the non-bonded regions of the continuous elastic laminate to form continuous lengths of leg gasketing assemblies having elastic regions and deactivated regions. The continuous lengths of leg gasketing assemblies may then bonded to a continuous topsheet substrate. Bonds, referred to herein as tack-down bonds, may then be applied to bond portions of the leg gasketing assemblies in the non-bonded regions with the topsheet substrate. The topsheet substrate may be combined with other components to form a continuous length of absorbent articles. And discrete diapers may then be formed by separating the continuous absorbent articles with a final knife cut. It is to be appreciated that the methods herein may be configured to perform assembly operations in various different orders. For example, in some embodiments, the elastic laminate may be combined with the topsheet substrate before severing the strands in the non-bonded regions.

It is to be appreciated that the elastic laminate can be formed in various ways. For example, in some embodiments, the first continuous substrate layer may be formed from a first continuous substrate, and the second continuous substrate layer may be formed from a second continuous substrate. In other embodiments, the first continuous substrate layer and/or the second continuous substrate layer may be formed by folding a portion of a single continuous substrate onto another portion of the single continuous substrate.

The processes and apparatuses discussed herein may be used to assemble elastic laminates with various types of substrate configurations, some of which may be used in the manufacture of different types of absorbent articles. To help provide additional context to the subsequent discussion of the process embodiments, the following provides a general description of absorbent articles in the form of diapers that include elastic laminates that may be assembled in accordance with the methods disclosed herein. Although the methods herein are discussed below in the context of manufacturing leg gasketing assemblies for absorbent articles, it is to be appreciated that the assembly methods herein may be configured to manufacture various types of substrates having intermittently spaced elastic and inelastic regions.

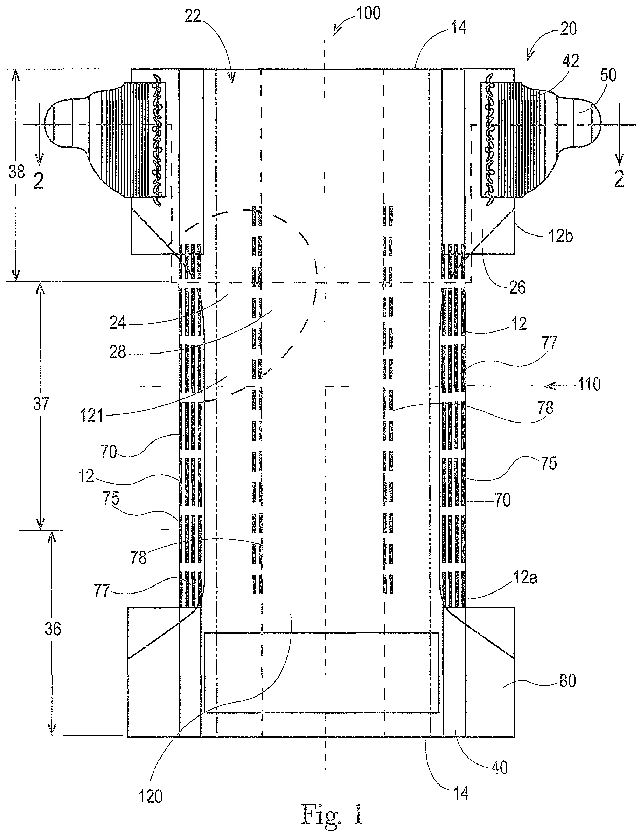

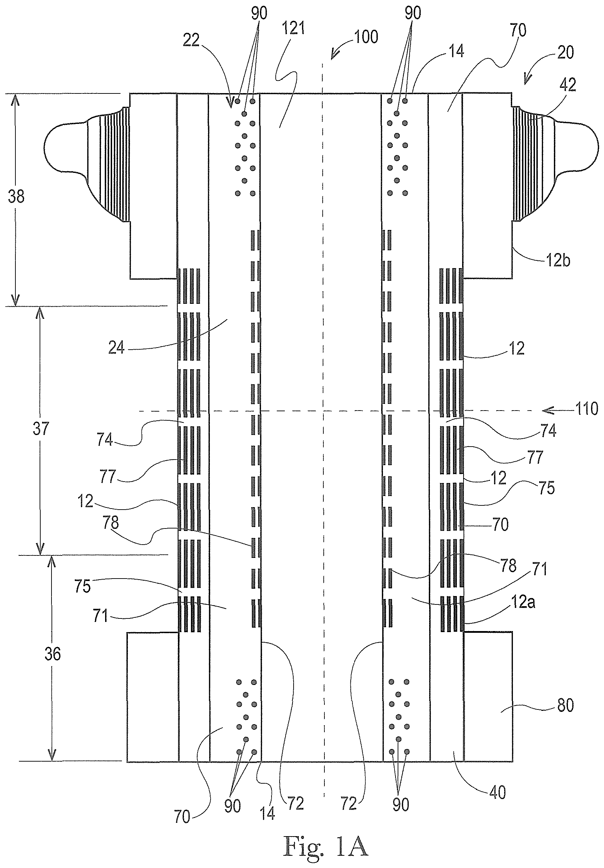

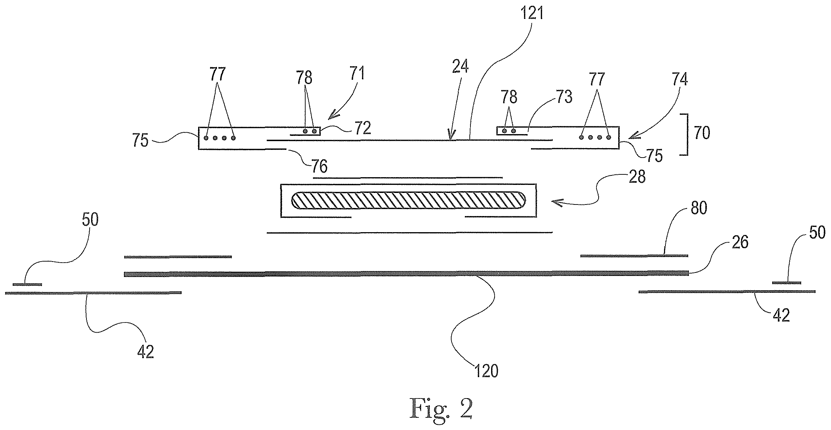

Figure 1 is a plan view of an exemplary, non-limiting embodiment of an absorbent article 20 in a flat, uncontracted state (*i.e.,* without elastic induced contraction) with the garment-facing surface 120 of the absorbent article 20 oriented toward the viewer. And Figure 1A is a plan view of the absorbent article 20 in a flat, uncontracted state (*i.e.,* without elastic induced contraction) with body-facing surface 121 of the absorbent article 20 oriented toward the viewer. Figure 2 is a cross-sectional view of the diaper 20 shown in Figure 1. With reference to Figures 1-2, the absorbent article 20 includes a longitudinal centerline 100 and a lateral centerline 110. The absorbent article 20 may comprise a chassis 22. The absorbent article 20 and chassis 22 are shown to have a first waist region 36, a second waist region 38 opposed to the first waist region 36, and a crotch region 37 located between the first waist region 36 and the second waist region 38. The waist regions 36 and 38 generally comprise those portions of the absorbent article 20 which, when worn, encircle the waist of the wearer. The waist regions 36 and 38 may include elastic elements such that they gather about the waist of the wearer to provide improved fit and containment. The crotch region 37 is that portion of the absorbent article 20 which, when the absorbent article 20 is worn, is generally positioned between the legs of the wearer.

The outer periphery of chassis 22 is defined by longitudinal edges 12 and lateral edges 14. The longitudinal edges 12 may be subdivided into a front longitudinal edge 12a, which is the portion of the longitudinal edge 12 in the first waist region 36, and a rear longitudinal edge 12b, which is the portion of the longitudinal edge 12 in the rear waist region 38. The chassis 22 may have opposing longitudinal edges 12 that are oriented generally parallel to the longitudinal centerline 100. In some embodiments, the longitudinal edges 12 may be curved or angled to produce, for example, an "hourglass" shape diaper when viewed in a plan view. The chassis 22 may have opposing lateral edges 14 that are oriented generally parallel to the lateral centerline 110.

The chassis 22 may comprise a liquid permeable topsheet 24, a backsheet 26, and an absorbent core 28 between the topsheet 24 and the backsheet 26. The absorbent core 28 may have a body-facing surface and a garment facing-surface. The topsheet 24 may be joined to the core 28 and/or the backsheet 26. The backsheet 26 may be joined to the core 28 and/or the topsheet 24. It should be recognized that other structures, elements, or substrates may be positioned between the core 28 and the topsheet 24 and/or backsheet 26. In some embodiments, the chassis 22 comprises the main structure of the absorbent article 20 with other features added to form the composite diaper structure. It is to be appreciated that the topsheet 24, the backsheet 26, and the absorbent core 28 may be assembled in a variety of configurations, such as for example, described generally in U.S. Patent Nos. 3,860,003; 5,151,092; 5,221,274; 5,554,145; 5,569,234; 5,580,411; and 6,004,306.

It is to also be appreciated that a portion or the whole of the diaper 20 may also be made laterally extensible. The additional extensibility may help allow the diaper 20 to conform to the body of a wearer during movement by the wearer. The additional extensibility may also help, for example, allow the user of the diaper 20 including a chassis 22 having a particular size before extension to extend the front waist region 36, the back waist region 38, or both waist regions of the diaper 20 and/or chassis 22 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper 20 may include a backsheet 26. The backsheet 26 may also define the outer, garment facing surface 120 of the chassis 22. The backsheet 26 may be impervious to fluids (e.g., menses, urine, and/or runny feces) and may be manufactured from a thin plastic film, although other flexible liquid impervious materials may also be used. The backsheet 26 may prevent the exudates absorbed and contained in the absorbent core from wetting articles which contact the diaper 20, such as bedsheets, pajamas and undergarments. The backsheet 26 may also comprise a woven or nonwoven material, polymeric films such as thermoplastic films of polyethylene or polypropylene, and/or a multi-layer or composite materials comprising a film and a nonwoven material (e.g., having an inner film layer and an outer nonwoven layer). The backsheet may also comprise an elastomeric film. An example backsheet 26 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385, X15306, X10962, and X10964. The backsheet 136 may also be embossed and/or matte-finished to provide a more clothlike appearance. Further, the backsheet 26 may permit vapors to escape from the absorbent core (i.e., the backsheet is breathable) while still preventing exudates from passing through the backsheet 26. The size of the backsheet 26 may be dictated by the size of the absorbent core 28 and/or particular configuration or size of the diaper 20.

Also described above, the diaper 20 may include a topsheet 24. The topsheet 24 may also define all or part of the inner, body facing surface 121 of the chassis 22. The topsheet 24 may be compliant, soft feeling, and non-irritating to the wearer's skin. It may be elastically stretchable in one or two directions. Further, the topsheet 24 may be liquid pervious, permitting liquids (e.g., menses, urine, and/or runny feces) to penetrate through its thickness. A topsheet 24 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 24 includes fibers, the fibers may be spunbond, carded, wet-laid, meltblown, hydroentangled, or otherwise processed as is known in the art.

Topsheets 24 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539.

As mentioned above, the diaper 20 may also include an absorbent core 28 that is joined to the chassis 22. The absorbent core 28 may additionally include one or more absorbent cores or absorbent core layers. The absorbent core 28 may be at least partially disposed between the topsheet 24 and the backsheet 26 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735; 4,888,231; 5,137,537; 5,147,345; 5,342,338; 5,260,345; 5,387,207; 5,397,316; and 5,625,222.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprises primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 and 2004/0097895.

As shown in Figures 1-2, the absorbent article 20 may include front ears 40 and/or back ears 42. The ears 40, 42 may be extensible, inextensible, elastic, or inelastic. The ears 40, 42 may be formed from nonwoven webs, woven webs, knitted fabrics, polymeric and elastomeric films, apertured films, sponges, foams, scrims, and combinations and laminates thereof. In certain embodiments the ears 40, 42 may be formed of a stretch laminate such as a nonwoven/elastomeric material laminate or a nonwoven/elastomeric material/nonwoven laminate. Stretch laminates may be formed by any method known in the art. For example, the ears 40, 42 may be formed as a zero strain stretch laminate, which includes at least a layer of non-woven material and an elastomeric element. The elastomeric element is attached to the layer of non-woven material while in a relaxed or substantially relaxed state, and the resulting laminate is made stretchable (or more stretchable over a further range) by subjecting the laminate to an activation process which elongates the nonwoven layer permanently, but the elastomeric element temporarily. The nonwoven layer may be integral with at least a portion of the chassis 22, in which case the elastomeric element may be attached to the nonwoven layer and the nonwoven/elastomeric element laminate is subsequently activated. Alternatively, the nonwoven layer may be a separate component, in which case the elastomeric element is attached to the nonwoven layer to form the laminate, which is then coupled to the main portion. If one or more layers of the side panel are provided separately, the laminate may be activated either before or after attachment to the main portion. The zero strain activation processes is further disclosed in U.S. Patent Nos. 5,167,897 and 5,156,793. A suitable elastic ear may be an activated laminate comprising an elastomeric film (such as is available from Tredegar Corp, Richmond, VA, as supplier code X25007) disposed between two nonwoven layers (such as is available from BBA Fiberweb, Brentwood, TN as supplier code FPN332).

The ears 40, 42 may be in the form of a discrete ear that is formed as separate element which is joined to the chassis 22. The ears may also be in the form of an integral ear that is a portion of the chassis 22 that projects laterally outward from the longitudinal edge 12. The integral ear may be formed by cutting the chassis form to include the shape of the ear projection.

The absorbent article 20 may also include a fastening system 50. When fastened, the fastening system 50 interconnects the first waist region 36 and the rear waist region 38 resulting in a waist circumference that may encircle the wearer during wear of the absorbent article 20. The fastening system 50 may comprise a fastener such as tape tabs, hook and loop fastening components, interlocking fasteners such as tabs & slots, buckles, buttons, snaps, and/or hermaphroditic fastening components, although any other known fastening means are generally acceptable. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. The fastening system 50 may also provide a means for holding the article in a disposal configuration as disclosed in U.S. Pat. No. 4,963,140. The fastening system 50 may also include primary and secondary fastening systems, as disclosed in U.S. Pat. No. 4,699,622. The fastening system 50 may be constructed to reduce shifting of overlapped portions or to improve fit as disclosed in U.S. Patent Nos. 5,242,436; 5,499,978; 5,507,736; and 5,591,152.

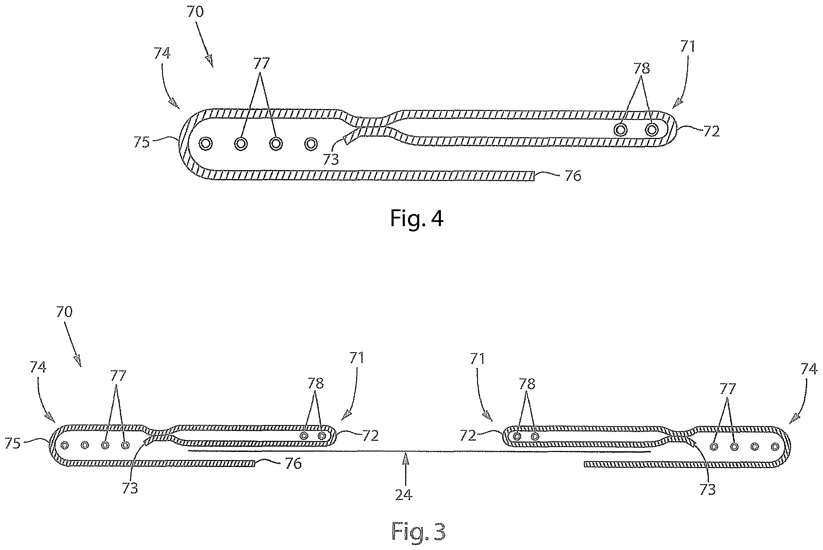

As previously mentioned and as shown in Figures 1-4, the absorbent article 20 may include a leg gasketing assembly 70. Figures 3 and 4 depict schematic cross section views of exemplary leg gasketing assemblies 70. The leg gasketing assemblies 70 may comprise an inner barrier leg cuff 71 (referred to herein as an "inner leg cuff" or "inner cuff") and an outer barrier leg cuff (referred to herein as an "outer leg cuff" or "outer cuff"). The inner leg cuff may include an inner cuff folded edge 72 and an inner cuff material edge 73. And the outer cuff 74 may include an outer cuff folded edge 75 and an outer cuff material edge 76. In some embodiments, the outer cuff material edge 76 is disposed laterally inboard the inner cuff material edge 73. In some embodiments, the leg gasketing assemblies 70 may extend from the first waist region 36 to the second waist region 38. And in some embodiments, the leg gasketing assemblies 70 may extend from the lateral edge 14 in the first waist region 36 to the lateral edge 14 in the second waist region 38. In addition, the leg gasketing assemblies may be joined with the topsheet 24 and/or backsheet 26 between the inner cuff folded edge 72 and the outer cuff folded edge 75.

As shown in Figure 1A, the absorbent article 20 may also include tack-down bonds 90 that bond portions of the inner cuffs 71 to the topsheet 24 in the first waist region 36 and/or second waist region 38. The tack-down bonds 90 may help hold distal regions of the inner cuffs 71, such as adjacent the inner cuff folded edge 72, against the topsheet 24. It is to be appreciated that in some embodiments, tack-down bonds may be applied to either or both the inner cuffs 71 and the outer cuffs 74 and topsheet 24 in the first waist region 36 and/or second waist region 38.

As shown in Figures 1-4, the outer leg cuff 74 may include elastic members 77 positioned in a lateral array between the outer cuff folded edge 75 and outer cuff material edge 76. As discussed below in more detail, the elastic members 77 may be in the form of elastic strands. It is to be appreciated that the outer leg cuff 74 may include various quantities of elastic members. For example, in some embodiments, the outer leg cuff 74 may include two, three, four, five, six, or more elastic members 77. As shown in Figures 3 and 4, the elastic members 77 may be disposed between the outer cuff folded edge 75 and the inner cuff material edge 73.

With continued reference to Figures 1-4, the inner barrier leg cuff 71 may include an array of elastic members 78 adjacent the inner cuff folded edge 72. As discussed below in more detail, the elastic members 77 may be in the form of elastic strands.. It is to be appreciated that the inner leg cuff 71 may include various quantities of elastic members. For example, in some embodiments, the inner leg cuff 71 may include one, two, three, four, five, or more elastic members 78. It should also be appreciated that the outer leg cuff 74 and the inner leg cuff 71 may include the same or different quantities of elastic members 77, 78. As shown in Figures 3 and 4, the elastic members 78 may be disposed between the inner cuff folded edge 72 and the outer cuff material edge 76.

It is to be appreciated that the elastic members 77, 78 may be located various distances with respect to various features of the leg gasketing assembly and/or may be located various distances with respect to each other. For example, in some embodiments, some of the elastic members 77 may separated from each other by 1mm, 2mm, 3mm, 3.5mm, 4mm, or more. And for example, in some embodiments, some or all the elastic members 78 may separated from each other by 1mm, 2mm, 3mm, 3.5mm, 4mm, or more.

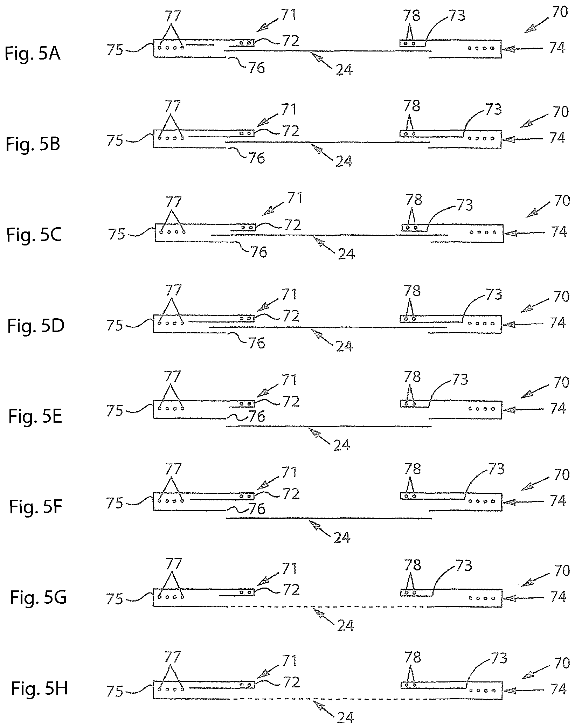

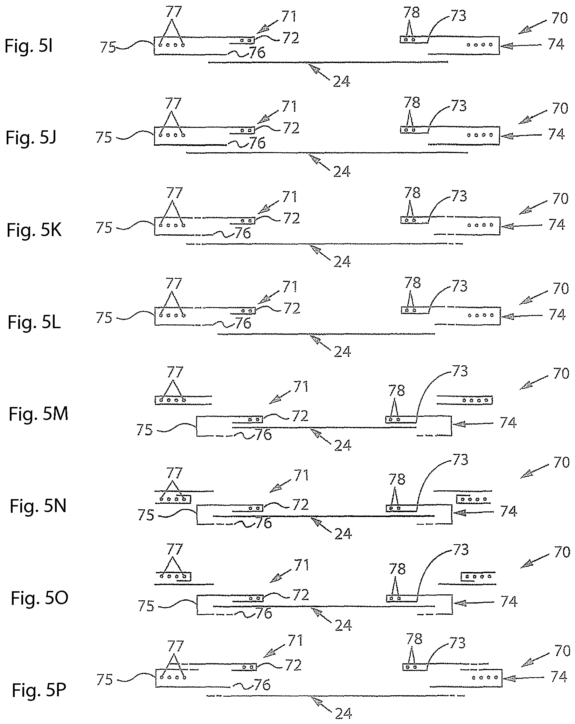

As shown in Figures 3 and 4, in some embodiments, the elastic members 77 may be located between the inner cuff material edge 73 and the outer cuff folded edge 75. And in some embodiments, the elastic members 78 may be located between the outer cuff material edge 76 and the inner cuff folded edge 72. In some embodiments, an additional material may be located between the inner cuff material edge 73 and the outer cuff material edge 76; and such material may include a topsheet 24; opacity strengthening patch 80; backsheet 28; core 26; or any other material positioned in the leg gasketing assembly 70. For example, Figure 3 shows a portion of a topsheet 24 positioned between the inner cuff material edge 73 and the outer cuff material edge 76. Figures 5A-5P show addition examples of cross sectional views of various leg gasketing assembly 70 and topsheet 24 arrangements.

As preiviously mentioned, the diaper 20 may include an opacity strengthening patch 80. The opacity strengthening patch 80 may be connected to the leg gasketing assembly 70, the polymeric film layer, or the backsheet 26. The opacity strengthening patch 80 may be postioned between the backsheet 26 and leg gasketing assemblies 70 in either the first waist region 36, the second waist region 38, or both the first waist region 36 and the second waist region 38 of the article; the opacity strengthening patch 80 may overlap at least one of the leg gasketing asembly 70 or the polymeric film layer using various techniques, such as glue, mechanical bonds, thermal bonds, or the like. The opacity strengthening patch may help prevent the article from extending excessively during application and wearing; and may also provide opacity at the sides and waist to help prevent the skin of the user from showing through the article. Thus, the patch 80 may be located at any portion of the chassis where strength and opacity is desirable. In some embodiments, the opacity strengthening patch may include materials having a basis weight of at least about 10gsm, at least about 15gsm, or at least about 25gsm.

As discussed in more detail below, the leg gasketing assembly 70 may be constructed from a single substrate or web of material. In some embodiments, the substrate may include a first material corresponding with the inner barrier leg cuff 71 and a second material corresponding with the outer cuff 74. The first and second materials may overlap and may be joined together along a longitudinal edge of each material in various ways.

It is to be appreciated that the leg gasketing assembly 70 may also be constructed from various types of materials. For example, the leg gasketing assembly 70 may be made from a substantially liquid impervious material, such as for example, an SMS nonwoven, SMMS nonwoven material, or a nonwoven component layer comprising "N-fibers". Various nonwoven fabric webs may comprise spunbond, meltblown, spunbond ("SMS") webs comprising outer layers of spunbond thermoplastics (e.g., polyolefins) and an interior layer of meltblown thermoplastics. In some embodiments, the leg gasketing assembly 70 may include a nonwoven component layer having fine fibers ("N-fibers") with an average diameter of less than 1 micron and (an "N-fiber layer") may be added to, or otherwise incorporated with, other nonwoven component layers to form a nonwoven web of material. In some embodiments, the N-fiber layer may be used to produce a SNS nonwoven web or SMNS nonwoven web, for example.

In some embodiments, the leg gasketing assembly 70 may include a first nonwoven component layer comprising fibers having an average diameter in the range of about 8 microns to about 30 microns, a second nonwoven component layer comprising fibers having a number-average diameter of less than about 1 micron, a mass-average diameter of less than about 1.5 microns, and a ratio of the mass-average diameter to the number-average diameter less than about 2, and a third nonwoven component layer comprising fibers having an average diameter in the range of about 8 microns to about 30 microns. The second nonwoven component layer is disposed intermediate the first nonwoven component layer and the third nonwoven component layer. In some embodiments, the leg gasketing assembly substrate may have various basis weights, such as for example, about 10gsm; 13 gsm; 15gsm; or 18 gsm.

The N-fibers may be include a polymer, for example, selected from polyesters, including PET and PBT, polylactic acid (PLA), alkyds, polyolefins, including polypropylene (PP), polyethylene (PE), and polybutylene (PB), olefinic copolymers from ethylene and propylene, elastomeric polymers including thermoplastic polyurethanes (TPU) and styrenic blockcopolymers (linear and radial di- and tri-block copolymers such as various types of Kraton), polystyrenes, polyamides, PHA (polyhydroxyalkanoates) and e.g. PHB (polyhydroxubutyrate), and starch-based compositions including thermoplastic starch, for example. The above polymers may be used as homopolymers, copolymers, e.g., copolymers of ethylene and propylene, blends, and alloys thereof. The N-fiber layer may be bonded to the other nonwoven component layers by any suitable bonding technique, such as the calender bond process, for example, also called thermal point bonding.

The inner barrier cuff 71 and/or outer cuff 74 may be treated, in full or in part, with a lotion, as described above with regard to topsheets, or may be fully or partially coated with a hydrophobic surface coating as detailed in U.S. Patent No. 7,626,073. Hydrophobic surface coatings usefully herein may include a nonaqueous, solventless, multicomponent silicone composition. The silicone composition includes at least one silicone polymer and is substantially free of aminosilicones.

It is to be appreciated that the leg gasketing assemblies may be bonded to various components of the diaper 20 in various locations. For example, in some embodiments, the leg gasketing assembly may be spaced laterally inward of the chassis edge 12 by about 10mm, about 20 mm, or about 30mm. In some embodiments, the laterally outboard edge of the chassis 12 may be defined by a lateral edge of the outer leg cuff 74. In some embodiments, the backsheet 26 may be spaced laterally inward of the outer cuff edge by about 10mm; about 20mm; about 30mm; or about 40mm. In some embodiments, the laterally outboard edge of the leg gasketing assembly 70 may be disposed laterally inboard at least a portion of the longitudinal edge of the article in at least one of the waist regions. Thus, in one embodiment, the front ears 40 and/or back ears 42 extend laterally outward further than the leg gasketing assembly 70. It is also to be appreciated that the leg gasketing assembly 70 may be connected with the diaper in various ways. For example, in some embodiments, the leg gasketing assembly may be joined to the topsheet 24 and/or backsheet 26 with a slot coated adhesive. Such slot coated adhesive may be applied in various quantities. For example, in some embodiments, at least about 12gsm, 15gsm, 20gsm, 25gsm, 40gsm, or 60gsm of adhesive may be applied. In some embodiments, the leg gaseting assembly may be joined with the topsheet and/or backsheet by spiral adhesive sprays.

As previously mentioned, the methods according to the present disclosure may be utilized to assemble various components of absorbent articles in the form of diapers 20. For example, Figure 6 shows a schematic view of a converting apparatus 300 adapted to manufacture diapers 20. The method of operation of the converting apparatus 300 may be described with reference to the various components of diapers 20 described above and shown in Figures 1-5P. Although the following description of methods are provided in the context of the diaper 20, such as shown in Figures 1-5P, it is to be appreciated that various embodiments of diapers can be manufactured according the methods disclosed herein, such as for example, the absorbent articles disclosed in U.S. Patent Nos. 7,569,039 and 5,745,922; U.S. Patent Publication Nos. 2005/0107764A1, 2012/0061016A1, and 2012/0061015A1, which are all hereby incorporated by reference herein.

As described in more detail below, the converting apparatus 300 shown in Figure 6 operates to assemble continuous lengths of leg gasketing assemblies 70 advancing in a machine direction MD, which are combined with a continuous length of an advancing topsheet substrate 24. Distal end portions of the leg gasketing assemblies 70 are then bonded to the topsheet substrate 24. The combined leg gasketing assemblies 70 and topsheet substrate 24 are then combined with a continuous length of an advancing backsheet substrate 26 and other diaper components, such as cores 28 and/or ears 40, 42 to create a continuous length of absorbent articles 20a. The continuous length of absorbent articles 20a are then subject to a final knife cut to create discrete diapers 20.

As shown in Figures 6 and 6A, two continuous lengths of leg cuff substrates 400 and inner cuff elastic strands 78 are advanced in a machine direction MD and combined at roll 302. More particularly, the leg cuff substrates 400 each include a first surface 401a and an opposing second surface 401b, and inner cuff elastic strands 78 are combined with first surfaces 401a of the leg cuff substrates 400. Before being combined at roll 302, the inner cuff elastic strands 78 are stretched in the machine direction MD. As shown in Figures 6, 6A, 6A1, and 6A2, from the roll 302, the combined elastics 78 and the cuff substrates 400 advance to a folding apparatus 304 adapted to fold a portion of the first surface 401a of each cuff substrate 400 onto another portion of the first surface 401a such that the elastics 78 are intermittently bonded between the folded portions of the cuff substrates 400. In the illustrated arrangement, adhesive 306a may be applied intermittently to the elastic strands 78 and/or the continuous lengths of cuff substrates 400 before entering the folder 304. Figures 6A, 6A1, and 6A2 shows an arrangement wherein each cuff substrate 400 includes opposing first and second longitudinal edges 402a, 402b and laterally opposed first and second edge regions 400a, 400b separated by a central region 400c. And the inner cuff elastics 78 are combined with the first edge region 400a of the first surface 401a of each cuff substrate 400. The folder 304 folds each first edge region 400a onto itself and/or the central region 400c. As shown in Figure 6A2, adhesive 306b may also be applied to the leg cuff substrate 400 adjacent the first longitudinal edge 402a. As such, the inner cuff elastics 78 are sandwiched between the first surface 401a of the opposing edge region 400a and the first surface 401a of itself and/or the central region 400c. Thus, the first longitudinal edge 402a of the leg cuff substrate 400 may correspond with the inner material cuff edge 73 discussed above. And the fold line in the cuff substrate 400 created by the folder 304 may correspond with the inner cuff folded edge 72. It is to be appreciated that other types of bonds may be used instead of or in combination with adhesive 306b shown in Figure 6A2, such as for example, dynamic mechanical bonds such as disclosed in U.S. Patent No. 4,919,738.

With continued reference to Figure 6, from the folder 304, the combined elastics 78 and the cuff substrates 400 advance to roll 308 and combined with outer cuff elastic strands 77. More particularly, the outer cuff elastic strands 77 are combined with first surfaces 401a of the leg cuff substrates 400. Before being combined at roll 308, the outer cuff elastic strands 77 are stretched in the machine direction MD. As shown in Figures 6, 6B, 6B 1, and 6B2, from the roll 308, the combined outer cuff elastics 77 and the cuff substrates 400 advance to a folding apparatus 310 adapted to fold a portion of the first surface 401a of each cuff substrate 400 onto another portion of the first surface 401a such that the outer cuff elastics 77 are intermittently bonded between the folded portions of the cuff substrate 400. In the illustrated arrangement, adhesive 306a may be applied intermittently to the elastic strands 77 and/or the continuous length of cuff substrate 400 before entering the folder 310. As shown in Figures 6B, 6B1, and 6B2, the elastics 78 may be combined with the second edge region 400b of the first surface 401a of each cuff substrate 400. The folder 310 folds each second edge region 400b onto itself and/or the central region 400c to create first and second elastic laminates 403. As shown in Figure 6B2, adhesive 306b may also be applied to the leg cuff substrate 400 adjacent the second longitudinal edge 402b. As such, the outer cuff elastics 77 are sandwiched between the first surface 401a of the second edge region 400b and the first surface 401a of itself and/or the central region 400c. Thus, the second longitudinal edge 402b of the leg cuff substrate 400 may correspond with the outer material cuff edge 75 discussed above. And the fold line in the cuff substrate 400 created by the folder 310 may correspond with the outer cuff folded edge 75. Further, as shown in Figures 6B and 6B2, each elastic laminate includes a first side 406a and an opposing second side 406b, wherein the second side 406b includes the first and second longitudinal edges 402a, 402b. As such, the first side 406a may define the first continuous substrate layer, and the second side 406b may define the second continuous substrate layer. In addition, in some embodiments, the first side 406a may correspond with a body facing surface of the leg gasketing assemblies 70, and the opposing second side 406b may correspond with a garment facing surface of the leg gasketing assemblies 70. It is to be appreciated that other types of bonds may be used instead of or in combination with adhesive 306b shown in Figure 6B2, such as for example, dynamic mechanical bonds such as disclosed in U.S. Patent No. 4,919,738.

It is to be appreciated that various sized portions of the cuff substrate 400 may be folded in various ways. For example, as shown in Figures 6B and 6B2, the cuff substrate 400 is folded such that the first longitudinal edge 402a and the second longitudinal edge 402b are positioned adjacent to each other and in some embodiments, may abut each to other. In other embodiments, the cuff substrate 400 may be folded so as to define a gap or space between the first longitudinal edge 402a and the second longitudinal edge 402b. In yet other embodiments, the cuff substrate 400 may be folded such that the first longitudinal edge 402a and/or second longitudinal edge 402b are in positioned in an overlapping arrangement. For example, the second longitudinal edge 402b may be folded over the first longitudinal edge 402a.

It should also be appreciated steps of combining the elastics 77, 78 with the cuff substrate 400 can be ordered in various ways. For example, the apparatus 300 may be configured to combine the outer cuff elastics 77 with the cuff substrate 400 before combining the inner cuff elastics 78 with the cuff substrate 400. In another example, the apparatus may be configured to combine the outer cuff elastics 77 and the inner cuff elastics 78 with the cuff substrate 400 at the same time. Although the embodiments described illustrate leg cuff assemblies 70 made from a single cuff substrate 400 combined with elastics 77, 78 and wherein the cuff substrate is folded onto itself, it is to be appreciated that the leg cuff assemblies can be formed by combining separate continuous lengths of cuff substrate material with elastic strands 77, 78. It should also be appreciated that the assembly process may be configured such a single substrate that is longitudinally slit along the machined direction and separated in the cross direction CD into two separate leg cuff substrates 400. In addition, the assembly process can be configured that the elastics 77, 78 are combined with the single substrate before or after the slitting operation.

As discussed above, the inner cuff elastic strands 78 and the outer cuff elastic strands 77 are intermittently bonded to the continuous lengths of cuff substrate 400 along the machine direction MD. For example, Figure 6A shows adhesive 306a (represented by cross-hatch areas) applied intermittently along the machine direction MD to the inner elastic strands 78 and the cuff substrate 400. And Figure 6B shows adhesive 306a (represented by cross-hatch areas) applied intermittently along the machine direction MD to the outer elastic strands 77 and the cuff substrate 400. As such, as shown in Figure 6B, the elastic laminates 403 may each include non-bonded regions 503 intermittently spaced between bonded regions 505 along the machine direction MD. Thus, the inner cuff elastic strands 78 and outer cuff elastic strands 77 are not bonded to leg cuff substrate 400 in the non-bonded regions 503. And the inner cuff elastic strands 78 and outer cuff elastic strands 77 are bonded to the leg cuff substrate 400 in the bonded regions 505. For the purposes of clarity, dashed lines 501 are shown in Figures 6B, 6C and others to represent example boundaries between the non-bonded regions 503 and the bonded regions 505. It is to be appreciated that the portions of the inner cuff elastics 78 and outer cuff elastics 77 intermittently bonded along the machine direction MD may be have the same or different lengths. As such, it is to be appreciated that such boundaries between the non-bonded regions 503 and the bonded regions 505 can also be curved, angled, and/or straight. Although the inner cuff elastic strands 78 and outer cuff elastic strands 77 are not bonded to the cuff substrate 400 in the non-bonded regions 503, adhesive may be applied in areas between the individual inner elastic strands 77, 78 to bond the folded portions of the cuff substrate 400 together in the non-bonded regions 503.

Referring back to Figure 6, from the folder 310, the continuous lengths of elastic laminates 403 advance in the machine direction MD to a cutting unit 312. The cutting unit 312 then intermittently deactivates the inner cuff elastics 78 and the outer cuff elastics 77 in the elastic laminates 403. More particularly, the cutting unit 312 may sever, cut, and/or break the cuff elastics 77, 78 in the non-bonded regions 503 of the elastics laminates 403. As shown in Figure 6C, severed ends 404 of the cuff elastics 77, 78 retract or snap back to the bonded regions 505, forming continuous lengths of leg gasketing assemblies 70a. As shown in Figures 6C and 6D, the leg gastketing assemblies 70a include elastic regions 505a and deactivated regions 503a. The elastic regions 505a of the leg gasketing assemblies 70a correspond with the bonded regions 505 of the elastic laminates 403. And the deactivated regions 503a of the leg gasketing assemblies 70a correspond with the non-bonded regions 503a of the elastic laminates 403. As shown in Figure 6C, the continuous lengths of leg gasketing assemblies also include continuous lengths of inner barrier leg cuff regions 71a and outer barrier leg cuff regions 74a. In some embodiments, the cutting unit 312 may be configured to sever only the elastics 77, 78 in the non-bonded regions 503 of the elastics laminates 403 without cutting through either cuff substrate 400. In other configurations, the cutting unit 312 may be configured to cut the elastics 77, 78 in the non-bonded regions 503 of the elastic laminates 403 while also cutting through the cuff substrate 400 one or both the opposing sides of the elastics 77, 78. It is also to be appreciated that the elastic laminates may be constructed by utilizing many of the various methods, apparatuses, and operations disclosed in U.S. Patent Application No. 13/434,984, filed on March 30, 2012, further identified by Attorney Docket No. 12390; U.S. Patent Application No. 13/435,036, filed on March 30, 2012, further identified by Attorney Docket No. 12391; U.S. Patent Application No. 13/435,063, filed on March 30, 2012, further identified by Attorney Docket No. 12392; U.S. Patent Application No. 13/435,247, filed on March 30, 2012, further identified by Attorney Docket No. 12393; and U.S. Patent Application No. 13/434,912, filed on March 30, 2012, further identified by Attorney Docket No. 12394; all of which are hereby incorporated by reference.

It is to be appreciated that the cutting unit 312 may be arranged to cut the elastics in different configurations. For example, in some embodiments, the cutting unit 312 may operate to sever only the inner cuff elastics 77 without severing the outer cuff elastics 78. In other embodiments, the cutting unit 312 may operate to sever only the outer cuff elastics 78 without severing the inner cuff elastics 77.

It is to be appreciated that various configurations of cutting units 312 can be used with the methods herein. Such cutting unit configurations may include features of the cutting knives/units disclosed, for example, in U.S. Patent Nos. 5,393,360; 7,708,849; 7,861,756; 7,777,094; U.S. Patent Application No. 13/434,912, filed on March 30, 2012, further identified by Attorney Docket Number 12394; and U.S. Patent Application No. 61/617,713, filed on March 30, 2012, further identified by Attorney Docket Number AA833FP-AF. As such, the cutting units may be configured with die knife, flexible blade, and/or compression roll features, and may also include additional features to control knife-anvil gaps and/or force.

Figures 7 and 8 show an example embodiment of a cutting unit 312 including a cutting roll 600 and an anvil roll 602. The cutting roll 600 is adapted to rotate around an axis of rotation 604 and defines an outer circumferential surface 606. And the anvil roll 602 is adapted to rotate around an axis of rotation 608 and defines an outer circumferential surface 610. The cutting roll 600 is adjacent to the anvil roll 602 and create a nip 603 defined by a minimum distance, D, between the outer circumferential surface 606 of the cutting roll 602 and the outer circumferential surface 610 of the anvil roll 602. As shown in Figures 7 and 8, the cutting roll 600 may also include one or more blades 612. Each blade 612 may have a proximal end portion 614 extending in a cross direction (CD) a length, L, along the outer circumferential surface 606 of the cutting roll 600. The blades 612 may define a dimension, H1, extending from the proximal end portion 614 to a distal edge 616. In addition, from the proximal end portion 614, the blades may extend radially outward from the outer circumferential surface 606 of the cutting roll 600 to the distal edge 616 by a distance, H. It is to be appreciated that the blades 612 may extend radially outward from the outer circumferential surface 606 to define an angle of 90 degrees or less, such as about 45 degrees, between the blade 612 and a tangential plane intersecting the proximal end portion 614 on the outer circumferential surface 606. As such, in some embodiments, H1 may be equal to H, and in some embodiments, H1 may be greater than H. As shown in Figures 6 and 7, the blades 612 may define a rectangular shape having a first surface 618 and an opposing second surface 620 separated by a thickness, t. The blades 612 may have a small thickness, t, relative to the distance, H1, such that blades 612 are flexible or bendable.

As shown in Figures 7-9C, the cutting unit 312 may be arranged such that the elastic laminates 403 advance in a machine direction MD to partially wrap around the outer circumferential surface 610 of the anvil roll 602. As the anvil roll 602 and the cutting roll 600 rotate, portions of the first surfaces 618 and the distal edges 616 of the blades 612 are moved into contact with the elastic laminates 403, such as shown in Figure 9A. As shown in Figures 9A-9C, the distance, H, of each blade 612 is greater than the distance, D, between the cutting roll 600 and the anvil roll 602. Thus, with reference to Figure 9B, as the blades 612 rotate though the nip 603 between the cutting roll 600 and the anvil roll 602, the blades 612 flex or bend inward along the second surface 620. As such, portions of the first surfaces 618 and/or the distal edges 616 of the blades 612 exert pressure on the first and second elastic laminates 400 to cut the elastics 77, 78 in the non-bonded regions 503. Referring now to Figure 8C, as the cutting roll 600 continues to rotate, the blades 612 move away from the nip 603 and straighten back out along the distance, H1, thus returning to the original blade shapes before entering the nip 603.

It is to be appreciated that the elastic laminates 403 may be arranged in various ways on the cutting unit 312. For example, as shown in Figures 7-9C, the elastic laminates 403 may advance in the machine direction MD to partially wrap around the rotating anvil roll 602 such that the first sides 406a are in contact with the outer circumferential surface 610 of the anvil roll 602. As such, the blades 612 of the rotating cutting roll 600 contact the second sides 406b of the elastic laminates 403 while advancing through the nip 603. It is to be appreciated that the elastic laminates 403 may be arranged such that either the first side 406a or the second side 406b is in contact with the outer circumferential surface 610 of the anvil roll 602. As discussed above, the cutting unit 312 shown in Figures 7-9C may be configured to sever only the elastics 77, 78 in the non-bonded regions 503 of the elastic laminates 403 without cutting through leg cuff substrate 400 material. In other configurations, the cutting unit 312 may be configured to cut the elastics 77, 78 in the non-bonded regions 503 of the elastic laminates 403 while also cutting through one or more layers of leg cuff substrate material 400.

It is to be appreciated that the cutting unit 312 may be configured with various quantities of blades having various shapes and orientations. For example, the cutting unit 312 shown in Figures 7-9C includes four blades 612a, 612b, 612c, 612d. The first and second blades 612a, 612b may be located 180 degrees apart from the third and fourth blades 612c, 612d on the outer circumferential surface 606 of the cutting roll 600. It is to be appreciated that the cutting roll may also be configured with various numbers of blades arranged circumferentially along the outer circumferential surface 606 of the cutting roll 600. The proximal end portions 614 of the first blade 612a and the second blade 612b may also be aligned with each other and with the axis of rotation 604 so as to extend in a straight line in the cross direction (CD) perpendicular to the machine direction (MD). Similarly, proximal end portions 614 of the third blade 612c and the fourth blade 612d may be aligned with each other and with the axis of rotation 604 so as to extend in a straight line in the cross direction (CD) perpendicular to the machine direction (MD). In addition, the first blade 612a and the second blade 612b, as well as the third and fourth blades 612c, 612d, may define different lengths, L, and may separated from each other by various distances in the cross direction CD. In addition, the blades 612 may be configured to cut the elastics 77, 78 simultaneously in the CD direction along in a substantially straight line. It is also to be appreciated that the cutting roll 600 may be configured with more than or less than two blades 612 aligned along the CD direction of outer circumferential surface 606 of the cutting roll 600. For example, in some embodiments, instead of having the first blade 612a and the second blade 612b, the cutting roll 600 may be configured with a single blade 612 extending along the CD direction for a length, L, on the outer circumferential surface 606.

With reference to Figures 6, 6D, 6D1, and 6D2, from the cutting unit 312, the first and second continuous lengths of leg gasketing assemblies 70a advance in the machine direction MD and are bonded with a continuous length of topsheet material substrate 24a at nip rolls 314. As shown in Figures 6D1 and 6D2, various types of bonds, such as for example adhesive 306, may be used to bond the first and second continuous lengths of leg gasketing assemblies 70a with the continuous length of topsheet material substrate 24a. From the nip rolls 314, the combined topsheet substrate 24a and leg gasketing assemblies 70a advance to a bonder unit 316. As shown in Figures 6, 6E, and 6E1, the bonder unit 316 bonds portions of the leg cuff assemblies 70a in the deactivated regions 503a to the topsheet substrate 70a. For example, as shown in 6E, and 6E1, the bonder unit 316 may operate to apply tack-down bonds 90 to the continuous lengths of inner barrier leg cuffs 71a in the deactivated regions 503a and the topsheet substrate 24a. It is to be appreciated that in some embodiments, the bonder unit 316 may operate to apply bonds to both or either the continuous inner barrier leg cuffs 71a and the outer barrier leg cuffs 74a. In addition, the tack-down bonds may also bond various layers of the inner barrier leg cuffs and/or outer barrier leg cuffs together. It should also be appreciated that various types of bonds 90 may be applied by the bonder unit 316, such as heat and pressure bonds, ultrasonic bonds, adhesive, and/or dynamic mechanical bonds such as disclosed in U.S. Patent No. 4,919,738, which is incorporated by reference herein.

It is to be appreciated that the nip rolls 314 illustrated in Figure 6 is a schematic representation of a process embodiment. It should be appreciated that other devices may used in place of the nip rolls 314, such as a S-wrapping arrangement of idlers that utilize web tension to combine the leg gasketing assemblies and the topsheet material substrate.

As shown in Figures 6 and 6E, the combined leg gasketing assemblies 70a and topsheet substrate 24a advancing from the bonder unit 316 may be combined with additional components and/or substrates to form a continuous length of absorbent articles 20a. Such additional components and/or substrates may include for example, a backsheet substrate 26a, absorbent cores 28, and/or ears 40, 42a. As shown Figures 6 and 6F, the continuous length of absorbent articles 20a may advance in the machine direction MD to a knife roll 318 where the continuous length of absorbent articles 20a are cut into discrete absorbent articles 20, such as shown in Figure 6G.

As previously mentioned, it is to be appreciated that the methods herein may be configured to perform assembly operations in various different orders. For example, the apparatus 300 in Figure 6 may be configured such that the elastic laminate 403 is combined with the topsheet substrate 24a before severing the elastic strands 77, 78 in the non-bonded regions 503. It should also be appreciated that the methods herein may be configured to bond with the leg cuff assemblies 70 with the absorbent articles 20 in various different locations, such as illustrated by the arrangements in Figures 5A-5P. Some embodiments may include separately formed inner cuffs and outer cuffs. As such, in some processes, the inner cuffs may be formed wherein the elastics are severed before being combined with a diaper or chassis having the outer leg cuffs already formed therein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for assembling disposable absorbent articles, the method comprising the steps of:
advancing a continuous topsheet substrate (24a) having a first surface and an opposing second surface in a machine direction (MD);
advancing a continuous leg cuff substrate (400) having a first surface and an opposing second surface in the machine direction, and defining a width in a cross direction, wherein the leg cuff substrate (400) includes opposing first and second longitudinal edges (402a, 402b) and laterally opposed first and second edge regions (400a, 400b) separated by a central region (400c);
advancing elastic strands (78) in the machine direction in a stretched state;
intermittently bonding the elastic strands (78) in the stretched state to the first surface of the leg cuff substrate (400);
folding the first edge region (400a) of the leg cuff substrate (400) onto a portion of either the first edge region (400a), the central region (400c), or the second edge region (400b) of the leg cuff substrate (400) such that the stretched elastic strands (78) are positioned between the first surface of the folded first edge region (400a) and the first surface of the portion of either the first edge region (400a), the central region (400c), or the second edge region (400b) to form an elastic laminate (403), the elastic laminate (403) including bonded regions (505) and non-bonded regions (503) intermittently spaced along the machine direction, wherein the elastic strands (78) are bonded to the leg cuff substrate (400) in the bonded regions (505), and wherein the elastic strands (78) are not bonded to the leg cuff substrate (400) in the non-bonded regions (503);
severing elastic strands (78) in the non-bonded regions (503) of the elastic laminate (403) to form a continuous length of leg gasketing assemblies (70a) having elastic regions (505a) intermittently spaced along the machine direction between deactivated regions (503a);
bonding the continuous length of leg gasketing assemblies (70a) with the continuous topsheet substrate (24a); and
cutting the continuous topsheet substrate (24a) and continuous length of leg gasketing assemblies (70a) along the cross direction in deactivated regions (503a).

2. The method of claim 1, wherein the step of severing the elastic strands (78) further comprises cutting the elastic strands (78) and the leg cuff continuous substrate (400) in the non-bonded regions (503).

3. The method of claim 1, wherein the step of severing the elastic strands (78) further comprises cutting the elastic strands (78) in the non-bonded regions (503) without cutting the leg cuff substrate (400).

4. The method according to any of the preceding claims, wherein the leg gasketing assemblies (70a) define inner leg cuffs (71a) and outer leg cuffs (74a), and further comprising the step of bonding the inner leg cuffs (71a) to the topsheet substrate (24a) in the deactivated regions (503).

5. The method according to any of the preceding claims, further comprising the step of bonding the continuous topsheet (24a) substrate to a continuous backsheet substrate (26a).

6. The method according to any of the preceding claims, wherein the step of severing the elastic strands (78) further comprises applying pressure to the leg cuff substrate (400) with a distal edge (616) of a blade (612).

7. The method according to any of the preceding claims, further comprising the step of advancing the elastic laminate (403) in the machine direction (MD) through a nip (603) defined between a cutting roll (600) and an anvil roll (602), the cutting roll(600) rotating around a first axis of rotation (604) and the anvil roll (602) rotating around a second axis of rotation (608), wherein the cutting roll (600) includes an outer circumferential surface (606), and wherein the anvil roll (602) includes an outer circumferential surface (610) defining a minimum distance D between the outer circumferential surface (606) of the cutting roll (600) and the outer circumferential surface (610), wherein the cutting roll (600) includes a blade (612) having a distal edge (616) defining a maximum distance H between the distal edge (616) and the outer circumferential surface (606) of the cutting roll (600), and wherein the H is greater than D.

8. The method of claim 7, further comprising the step of: bending the blade (612).

## Patentansprüche

1. Verfahren zum Zusammensetzen von Einweg-Absorptionsartikeln, wobei das Verfahren die folgenden Schritte umfasst:
Vorwärtsbewegen eines kontinuierlichen Oberschichtsubstrats (24a) mit einer ersten Oberfläche und einer entgegengesetzten, zweiten Oberfläche in einer Maschinenlaufrichtung (MD);
Vorwärtsbewegen eines kontinuierlichen Beinbündchensubstrats (400) mit einer ersten Oberfläche und einer entgegengesetzten, zweiten Oberfläche in der Maschinenlaufrichtung, und Definieren einer Breite in einer Querrichtung, wobei das Beinbündchensubstrat (400) einander entgegengesetzte, erste und zweite Längsrichtungsränder (402a, 402b) und einander lateral entgegengesetzte, zweite Randregionen (400a, 400b), die durch eine mittlere Region (400c) voneinander getrennt werden, aufweist;
Vorwärtsbewegen elastischer Stränge (78) in der Maschinenlaufrichtung in einem gedehnten Zustand;
intermittierendes Heften der elastischen Stränge (78) im gestreckten Zustand an die erste Oberfläche des Beinbündchensubstrats (400);
Falten der ersten Randregion (400a) des Beinbündchensubstrats (400) auf einen Abschnitt entweder der ersten Randregion (400a), der mittleren Region (400c) oder der zweiten Randregion (400b) des Beinbündchensubstrats (400), so dass die gedehnten elastischen Stränge (78) zwischen der ersten Oberfläche der gefalteten ersten Randregion (400a) und der ersten Oberfläche des Abschnitts entweder der ersten Randregion (400a), der mittleren Region (400c) oder der zweiten Randregion (400b) positioniert werden, um ein elastisches Laminat (403) zu bilden, wobei das elastische Laminat (403) gebundene Regionen (505) und nichtgebundene Regionen (503) aufweist, die intermittierend entlang der Maschinenlaufrichtung mit Abständen angeordnet sind, wobei die elastischen Stränge (78) in den gebundenen Regionen (505) an das Beinbündchensubstrat (400) gebunden sind, und wobei die elastischen Stränge (78) in den nichtgebundenen Regionen (503) nicht an das Beinbündchensubstrat (400) gebunden sind;
Durchtrennen der elastischen Stränge (78) in den nichtgebundenen Regionen (503) des elastischen Laminats (403), um ein kontinuierliches Stück aus Beinabdichtungsanordnungen (70a) mit elastischen Regionen (505a) zu bilden, die intermittierend in Abständen entlang der Maschinenlaufrichtung zwischen deaktivierten Regionen (503a) angeordnet sind;
Verbinden des kontinuierlichen Stücks aus Beinabdichtungsanordnungen (70a) mit dem kontinuierlichen Oberschichtsubstrat (24a); und
Durschneiden des kontinuierlichen Oberschichtsubstrats (24a) und des kontinuierlichen Stücks aus Beinabdichtungsanordnungen (70a) entlang der Querrichtung in deaktivierten Regionen (503a).

2. Verfahren nach Anspruch 1, wobei der Schritt des Durchtrennens der elastischen Stränge (78) ferner das Durchschneiden der elastischen Stränge (78) und des kontinuierlichen Beinbündchensubstrats (400) in den nichtgebundenen Regionen (503) umfasst.

3. Verfahren nach Anspruch 1, wobei der Schritt des Durchtrennens der elastischen Stränge (78) ferner das Durchschneiden der elastischen Stränge (78) in den ungebundenen Regionen (503) ohne Durchschneiden des Beinbündchensubstrats (400) umfasst.

4. Verfahren nach einem der vorstehenden Ansprüche, wobei die Beinabdichtungsanordnungen (70a) innere Beinbündchen (71a) und äußere Beinbündchen (74a) definieren, und ferner den Schritt des Bindens der inneren Beinbündchen (71 a) an das Oberschichtsubstrat (24a) in den deaktivierten Regionen (503) umfassend.

5. Verfahren nach einem der vorstehenden Ansprüche, ferner den Schritt des Verbindens des Substrats der kontinuierlichen Oberschicht (24a) mit einem kontinuierlichen Unterschichtsubstrat (26a) umfassend.

6. Verfahren nach einem der vorstehenden Ansprüche, wobei der Schritt des Durchtrennens der elastischen Stränge (78) ferner das Anwenden von Druck auf das Beinbündchensubstrat (400) mit einem distalen Rand (616) eines Messers (612) umfasst.

7. Verfahren nach einem der vorstehenden Ansprüche, das ferner den Schritt des Vorwärtsbewegens des elastischen Laminats (403) in Maschinenlaufrichtung (MD) durch einen Walzenspalt (603) umfasst, der zwischen einer Schneidwalze (600) und einer Ambosswalze (602) gebildet wird, wobei die Schneidwalze (600) um eine erste Drehachse (604) rotiert und die Ambosswalze (602) um eine zweite Drehachse (608) rotiert, wobei die Schneidwalze (600) eine Außenumfangsfläche (606) aufweist, und wobei die Ambosswalze (602) eine Außenumfangsfläche (610) aufweist, die einen Mindestabstand D zwischen der Außenumfangsfläche (606) der Schneidwalze (600) und der Außenumfangsfläche (610) definiert, wobei die Schneidwalze (600) ein Messer (612) mit einem distalen Rand (616) aufweist, der einen maximalen Abstand H zwischen dem distalen Rand (616) und der Außenumfangsfläche (606) der Schneidwalze (600) definiert, und wobei H größer als D ist.

8. Verfahren nach Anspruch 7, das ferner den folgenden Schritt umfasst: das Biegen des Messers (612).

## Revendications

1. Procédé d'assemblage d'articles absorbants jetables, le procédé comprenant les étapes consistant à :
faire avancer un substrat continu de feuille de dessus (24a) possédant une première surface et une deuxième surface opposée dans une direction de la machine (MD) ;
faire avancer un substrat continu de revers de jambe (400) possédant une première surface et une deuxième surface opposée dans la direction de la machine et définissant une largeur dans une direction croisée, dans lequel le substrat de revers de jambe (400) inclut des premier et deuxième bords longitudinaux opposés (402a, 402b) et des première et deuxième régions de bord latéralement opposées (400a, 400b) séparées par une région centrale (400c) ;
faire avancer des fils élastiques (78) dans la direction de la machine dans un état étiré ;
lier de façon intermittente les fils élastiques (78) dans l'état étiré, à la première surface du substrat de revers de jambe (400);
plier la première région de bord (400a) du substrat de revers de jambe (400) sur une partie de l'une ou l'autre parmi la première région de bord (400a), la région centrale (400c) ou la deuxième région de bord (400b) du substrat de revers de jambe (400) de telle sorte que les fils élastiques étirés (78) sont positionnés entre la première surface de la première région de bord pliée (400a) et la première surface de la partie de l'une ou l'autre parmi la première région de bord (400a), la région centrale (400c) ou la deuxième région de bord (400b) pour former un stratifié élastique (403), le stratifié élastique (403) incluant des régions liées (505) et des régions non liées (503) espacées de façon intermittente le long de la direction de la machine, dans lequel les fils élastiques (78) sont liés au substrat de revers de jambe (400) dans les régions liées (505), et dans lequel les fils élastiques (78) ne sont pas liés au substrat de revers de jambe (400) dans les régions non liées (503) ;
trancher les fils élastiques (78) dans les régions non liées (503) du stratifié élastique (403) pour former une longueur continue d'ensembles d'étanchéité de jambe (70a) comportant des régions élastiques (505a) espacées de façon intermittente le long de la direction de la machine entre les régions désactivées (503a) ;
lier la longueur continue d'ensembles d'étanchéité de jambe (70a) avec le substrat continu de feuille de dessus (24a) ; et
couper le substrat continu de feuille de dessus (24a) et la longueur continue d'ensembles d'étanchéité de jambe (70a) le long de la direction croisée dans les régions désactivées (503a).

2. Procédé selon la revendication 1, dans lequel l'étape consistant à trancher les fils élastiques (78) comprend en outre la découpe des fils élastiques (78) et du substrat continu de revers de jambe (400) dans les régions non liées (503).

3. Procédé selon la revendication 1, dans lequel l'étape consistant à trancher les fils élastiques (78) comprend en outre la découpe des fils élastiques (78) dans les régions non liées (503) sans couper le substrat de revers de jambe (400).

4. Procédé selon l'une quelconque des revendications précédentes, dans lequel les ensembles d'étanchéité de jambe (70a) définissent des revers de jambe internes (71 a) et des revers de jambe externes (74a), et comprenant en outre l'étape consistant à lier les revers de jambe internes (71 a) au substrat de feuille de dessus (24a) dans les régions désactivées (503).

5. Procédé selon l'une quelconque des revendications précédentes, comprenant en outre l'étape consistant à lier le substrat continu de feuille de dessus (24a) à un substrat continu de feuille de fond (26a).

6. Procédé selon l'une quelconque des revendications précédentes, dans lequel l'étape consistant à trancher les fils élastiques (78) comprend en outre l'application d'une pression au substrat de revers de jambe (400) avec un bord distal (616) d'une lame (612).

7. Procédé selon l'une quelconque des revendications précédentes, comprenant en outre l'étape consistant à faire avancer le stratifié élastique (403) dans la direction de la machine (MD) à travers une ligne de contact (603) définie entre un rouleau de coupe (600) et un contre-rouleau (602), le rouleau de coupe (600) tournant autour d'un premier axe de rotation (604) et le contre-rouleau (602) tournant autour d'un deuxième axe de rotation (608), dans lequel le rouleau de coupe (600) inclut une surface circonférentielle externe (606), et dans lequel le contre-rouleau (602) inclut une surface circonférentielle externe (610) définissant une distance minimale D entre la surface circonférentielle externe (606) du rouleau de coupe (600) et la surface circonférentielle externe (610), dans lequel le rouleau de coupe (600) inclut une lame (612) possédant un bord distal (616) définissant une distance maximale H entre le bord distal (616) et la surface circonférentielle externe (606) du rouleau de coupe (600), et dans lequel la distance H est plus grande que la distance D.

8. Procédé selon la revendication 7, comprenant en outre l'étape consistant à : plier la lame (612).
